# EUROPEAN PATENT APPLICATION

(11) **EP 1 314 360 A1**
(43) Date of publication of application: **28.05.2003**
(21) Application number: 02025284.7
(22) Date of filing: 12.11.2002
(51) Int. Cl.: A23L 1/236, A23L 2/60, C07K 5/06

(54) **Process for producing a stable aspartame slurry and testing method for determining its stability**

(30) Priority: 22.11.2001 JP 2001358280
(71) Applicant: Ajinomoto Co., Inc., Tokyo 104 (JP)
(72) Inventor: Tomiyama, Yasuyuki, c/o Ajinomoto Co., Inc., Kawasaki-ku, Kawasaki-shi, Kanagawa (JP); Kawauchi, Masato, c/o Ajinomoto Co., Inc., Kawasaki-ku, Kawasaki-shi, Kanagawa (JP)
(74) Representative: Strehl Schübel-Hopf & Partner

(57) **Abstract**

There is provided a stable aspartame (APM) slurry where APM particles are not precipitated, no flocculation takes place but the particles are homogeneously dispersed and physical preservation stability is further improved. In the manufacture of an APM slurry, the slurry is prepared in such a manner that, in case the particle size distribution of the slurry is measured by a particle size distribution meter which is able to determine the state of particle aggregation in the slurry in a nondestructive manner in a pH region suitable for microbiological preservation of food, a single peak is substantially shown within a range of 1000 µm or less, and the particle size of APM in the APM slurry is made 10 µm at the largest in terms of the median diameter whereupon the aimed stable. APM slurry may be manufactured. It is suitable as a liquid sweetener. It is also possible by the present invention to provide the stable APM slurry and a method for judging the same.

## Description

### BACKGROUND OF THE INVENTION

### 1. Field of the Invention

The present invention relates to a process for producing a stable APM slurry containing aspartame (hereinafter, abbreviated as "APM"), which is a sweetener of the amino acid type, in high concentrations, to a method for its judgement and to an APM slurry manufactured by usingsuch process and/or method for judgement.

By the present invention it is possible to prepare a very stable APM suspension which, therefore, is suitable as a liquid sweetener.

### 2. Description of the Related Art

An APM slurry in which aspartame (APM) is suspended has been used as a liquid sweetener. With regard to an art for the production of the APM slurry, there have been many reports on a method of manufacturing the APM slurry and for the improvement in chemical preservation stability of APM (refer to Japanese Patent Kokai Publications JP-A-59-31669, 59-31656 and 59-151848; International Patent Publication WO 95/15697; European Patent No. 102032; etc.) but there has been no report where the physical stability of APM slurry upon preservation is sufficiently solved.

Up to now, several investigations have been carried out concerning the physical storage stability of APM slurry.

For example, there is proposed a method where the particle size of APM is made small and the viscosity of the slurry as a whole is increased whereby the stability is improved without precipitation of the particles (refer to International Patent Publication WO 95/15697) but, in some viscosity ranges (especially when the set viscosity is high), there is the problem of decreased fluidity (handling property).

The precipitation behaviour of APM slurry was also investigated for improving the physical storage stability at a low viscosity and there was reported that by adjusting the particle size of APM to not more than 10 µm or, preferably, not more than 8 µm in terms of the median diameter at a relatively low viscosity (such as 100 mPa·s or lower) a stable slurry resulting in no precipitation is prepared (refer to Japanese Patent Kokai Publication JP-A-11-313636). However, even by that method, APM particles in the APM slurry are not homogeneously dispersed but unstable dispersions are often noted.

Therefore, there is a demand for an APM slurry where the APM particles in the APM slurry are homogeneously dispersed and, further, the physical storage stability is high.

### SUMMARY OF THE INVENTION

### 1.Problem to be solved by the Invention

In general, there are two methods for the crystallization in the manufacture of APM. One is a stirring crystallization where the crystallization with cooling is carried out under a stirring condition and another is a static crystallization where a crystallization with cooling is carried out under a non-stirring condition (refer to Japanese Patent Kokai Publication JP-A-58-177952). The APM crystals obtained by the static crystallization have a larger diameter of the short axis than the APM crystals obtained by the stirring crystallization. Accordingly, they are excellent in their handling property such as solid-liquid separation and physical powder property of the crystals. Therefore, the static crystallization is sometimes adopted as an industrial crystallization process. However, even in the case of the static crystallization, the resulting crystal sizes are different depending upon the presence of impurities such as salts. For example, although there is a difference more or less depending upon the crystallization condition, the crystal particle size when APM hydrochloride is neutralized at high temperature and then subjected to a static cooling (one-step crystallization) is about 6 µm in terms of a median diameter measured by a particle size distribution meter used for the measurement of the particle size of a powder. When the crystals are once separated, dissolved in water again and subjected to the static crystallization (two-step crystallization), the concentration of the impurities is extremely lowered whereby the median diameter of the resulting crystals becomes about 20 µm (although there are some differences depending upon the crystallization condition). When APM having a big particle size as such is used for a slurry of a low viscosity system, it is necessary to crush the APM particles for adjusting the particle size. In addition, since the crystals formed by the stirring crystallization are very fine as well, blocks and big lumps are rather apt to be formed by drying and, therefore, it is also necessary to grind them in such a case.

On the other hand, since an APM slurry is used for foods, its microbiological preservation stability is demanded. For such a purpose, there is a report on a method where sodium benzoate is added and, although the pH condition at that time is not described, the condition where sodium benzoate achieves a bacteriostatic effect is pH 4 or lower and thus it is presumed that the pH is made 4 or lower (refer to Japanese Patent Kokai Publication JP-A-11-313636).

However, in a slurry of a specific particle size (such as 10 µm or smaller in terms of the median diameter), no precipitation takes place while, in the pH region where the microbiological preservation stability as food is taken into consideration such as a decrease of the pH value to 4 or lower, flocculation (coagulation due to aggregation of particles) is generated and, because of their vigorous aggregation, that sometimes badly affects the fluidity. It has been found that, as a result of the generation of flocculation as such, a stable dispersion (APM slurry) is hardly available.

Under such circumstances, it is necessary to solve the problem of flocculation and to ensure that the APM particles are not precipitated but are homogeneously dispersed to give a very stable APM slurry.

Thus, the problem to be solved by the present invention is to provide a stable APM slurry in which APM particles are not precipitated but are homogeneously dispersed without flocculation in the pH region suitable for a microbiological preservation stability as food and thereby to further improve the physical preservation stability while, taking into consideration the fluidity, the APM slurry has a relatively low viscosity (such as 100 mPa·s or less) .

### 2. Means to solve Problem

In order to solve the above problem, the present inventors have firstly carried out intensive investigations for the generation of flocculation and, as a result, they have found that generation of flocculation is greatly dependent upon the correlation between the fact whether grinding of particles is done for adjusting the particles size as well as the ground particle size and the pH value of the system, that flocculation takes place when the dispersed state of the APM particles in the slurry is nonhomogeneous and that, more specifically, the APM particles are able to maintain a stable slurry if a micro aggregation reaction takes place in the slurry and the aggregation takes place homogeneously while, in case the aggregation takes place non-homogeneously, flocculation results. It has been further found that, when the particle size distribution of the APM slurry is measured using a particle size distribution meter (such as Lasentec D600L [FBRM] which is a monitoring system of the inline type manufactured by Lasentec; hereinafter, it will be sometimes abbreviated as "Lasentec") which is able to grasp the state of particle aggregation in the slurry in a nondestructive manner as a means for measuring the state of aggregation, a slurry which shows substantially a single peak has a stable physical property while that which shows a plurality of peaks generates flocculation. The present invention has been achieved on the basis of various findings as such.

Thus, the present invention relates to a process for producing a stable APM slurry which comprises adjusting the particle size distribution of an APM slurry in an edible dispersing medium in such way that its measurement by a particle size distribution meter, which is able to measure the state of particle aggregation in the slurry in a nondestructive manner in a pH region suitable for the microbiological preservation of food, results in a single peak within the range of 1000 µm or less, and adjusting the particle size of APM in the APM slurry to 10 µm at the largest in terms of the median diameter. (Hereinafter, this process will be sometimes referred to as "the process for the manufacture of APM slurry of the present invention").

In accordance with the process for producing an APM slurry of the present invention, it is possible to obtain the slurry in the pH region suitable for the microbiological preservation of food described above, preferably not higher than pH 4 or so, and more preferably, at a pH of from about 4 to 2. Further, with regard to the particle size of the APM described above, it is preferably adjusted to about 6 µm at the largest (not larger than 6 µ m) or, more preferably, about 3 µm at the largest (not larger than 3 µm) in terms of the median diameter. Particularly, in the process for the manufacture of APM slurry of the present invention, when the APM particles described above are dried and crushed (ground) by a common drying method, particularly by a gas stream dryer (such as Micron Drier manufactured by Hosokawa Micron K. K.) to adjust the particle size as described above as a result and by adjusting the particle size distribution it is possible to provide a stable slurry which shows the above described single peak for most of the part. Although it is preferable that the crystallization condition is selected so that the above dried substance is within a range of the aimed particle size as above (further adjustment of the particle size being unnecessary), it is also possible to further adjust the particle size (by a common grinding means or the like) when the size is more than the aimed particle size as above and particles of 6 µm or smaller in the particle size are to be prepared.

As another embodiment, the present invention relates to the stable APM slurry (hereinafter, it is sometimes called "the APM slurry of the present invention") which is produced or is obtainable by the process for producing APM slurry 'of the present invention.

As a still another embodiment, the present invention relates to a testing method for the judgement of a stable APM slurry wherein an APM slurry to be judged is subjected to the following steps '(hereinafter, it will be sometimes called "the method for the judgement of APM slurry of the present invention"):
(a) a step wherein the particle size distribution of the slurry is measured by a particle size distribution meter which is able to grasp the state of particle aggregation in the slurry in a nondestructive manner, and the numbers of the peaks shown in the range of not more than 1000 µm (1000 µm or less)are counted; and
(b) a step where, in the above step, the slurry which gives a substantially single peak is recognized and collected as a stable APM slurry.

The method for the judgement of APM slurry of the present invention is also able to judge an APM slurry which is in a pH region suitable for the microbiological preservation of food, preferably at a pH of about 4 or lower and, more preferably, at a pH of from about 4 to 2. It is also possible to judge an APM slurry which is prepared in such way that the particle size of APM in the above described APM slurry is preferably about 10 µm at the largest in terms of the median diameter, more preferably about 6 µm at the largest in terms of the median diameter and, still more preferably, about 3 µm at the largest in terms of the median diameter. Particularly, in the method for the judgement of APM slurry of the present invention, it is possible that the APM particles described above are dried and crushed by a common drying method, particularly by a gas stream dryer (such as Micron Drier manufactured by Hosokawa Micron K. K.) and subjected to a further particle size adjustment as described above if necessary and the prepared slurry is judged whereupon a stable APM slurry is prepared.

The present invention further relates to the stable APM slurry which is prepared or is able to be prepared by the method for the judgement of APM slurry of the present invention.

### BRIEF DESCRIPTION OF THE DRAWINGS

### [Fig. 1]

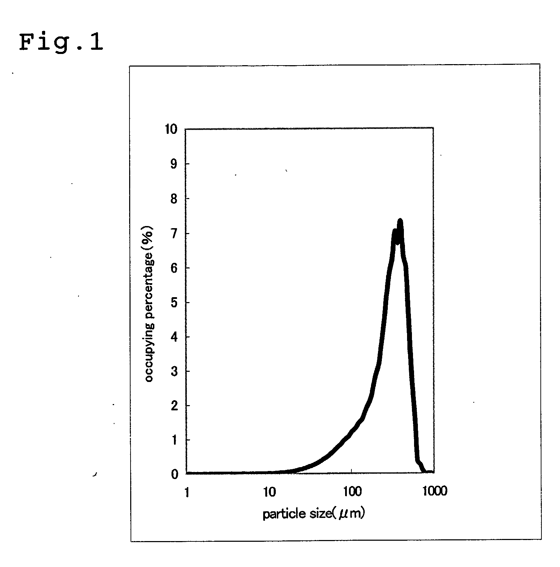

Fig. 1 shows the result of measurement of the particle size distribution of APM particles for the APM slurry of sample 1 and sample 3 by a Lasentec D600L [FBRM] of an inline type of monitoring system manufactured by Lasentec in which the ordinate shows an occupying percentage (%) while the abscissa shows the particle size (µm) of the APM particles.

### [Fig. 2]

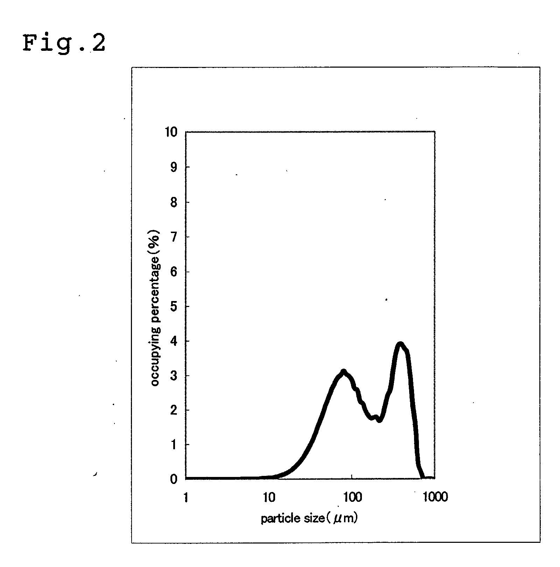

Fig. 2 shows the result of measurement of the particle size distribution of APM particles for the APM slurry of sample 2 by a Lasentec D600L [FBRM] of an inline type of monitoring system manufactured by Lasentec in which the ordinate shows an occupying percentage (%) while the abscissa shows the particle size (µm) of the APM particles.

### EMBODIMENTS FOR CARRYING OUT THE INVENTION

Hereafter, embodiments of the present invention will be illustrated.

The present invention includes several embodiments, i.e. a process for producing stable APM slurry, a method for its judgement and the stable APM slurry which is produced by such process/method. Incidentally, the illustration will be made mostly on the process for the production of the APM slurry of the present invention and on the method for the judgement thereof although the present invention is not limited thereto.

### (Process for the production of APM slurry of the present invention)

In the process for the production of APM slurry of the present invention, when the APM slurry is prepared by dispersing APM particles of about 10 µm at the largest, preferably about 6 µm at the largest and, more preferably, about 3 µm at the largest in an edible dispersing medium (such as water) within a pH region suitable for the microbiological preservation of food, a stable APM slurry is produced in such a manner that the particle size distribution of the slurry upon measurement by a particle size distribution meter which is able to determine the state of particle aggregation in the slurry in a nondestructive manner such as a Lasentec D600L [FBRM] which is an inline type of monitoring system manufactured by Lasentec shows substantially a single peak. Thus, when the slurry is prepared in such a manner that, when particle size distribution of the slurry is measured by the above described particle size distribution meter taking conditions such as particle size of the APM particles, pH value of the slurry and concentration thereof into consideration, there is obtained the pattern of Fig. 1 which will be shown later or, to be specific, a pattern showing substantially a single peak within a range of 1000 µm or less, then it is possible to manufacture the aimed stable APM slurry.

The term "showing substantially a single peak" covers not only cases where only one peak is clearly shown but also cases where, with regard to another peak which is present besides the clear first peak, recognition of the second peak is difficult, etc.

With regard to the particle size distribution meter described above, it is possible to use, for example, a Lasentec D600L [FBRM] which is a monitoring system of an inline type manufactured by Lasentec and, besides that, it is also possible to use not only a particle size distribution meter being able to directly determine the state of particle aggregation but also a particle size distribution meter being able to indirectly determine the state of particle aggregation that has been known already or will be developed in future.

On the other hand, with regard to the measurement of the particle size of the APM (median diameter) described above, it is possible to use particle size distribution meters (for the measurement of particle size of powder) which are commonly used for the measurement of particle size of the powder such as LA920 (manufactured by Horiba K.K.).

The APM slurry described above contains at least the APM and the edible dispersing medium (such as water) and, if necessary, it may further contain a pH adjusting agent. It is also possible to appropriately use a carrier (such as D-sorbitol) used for sweetener, auxiliary substances such as extender (bulking agent) and excipient and other component necessary for a liquid sweetener.

With regard to the pH value of the slurry, there is no particular limitation so far as it is a microbiologically safe pH region for food as mentioned already. Preferably, a pH value of about (approximately) 4 or lower and, more preferably, from about (approximately) 4 to 2 may be selected.

With regard to the concentration of the APM slurry described above, there is no particular limitation so far as the slurry state is maintained at room temperature. For example, there may be selected a concentration where APM in an amount of more than its solubility in the edible dispersing medium (such as water) within the pH region is added thereto. A more preferred adding amount is preferably about 3 to 15 g and, more preferably, about 5 to 10 g to 100 g of the edible dispersing medium such as water at room temperature.

In the process for the production of APM slurry of the present invention, there is no particular difficulty in preparing the slurry. However, when foams are mixed in the APM slurry during the preparation, the single peak is not achieved and hence flocculation takes place in the slurry. It is therefore necessary to prepare the slurry in such a manner that no foam is mixed therewith.

As mentioned already, the occurrence of flocculation is greatly dependent upon the correlation between the fact whether APM particles are ground for the adjustment of particle size as well as on the ground particle size and the pH value of the system. For example, when the particle size of APM in the APM slurry is about (approximately) 3 µm at the largest in terms of median diameter, a single peak as shown in the pattern of Fig. 1 is observed in most cases provided that mixing of foams is prevented. Accordingly, when a slurry is prepared so as not to mix the foams and when it is confirmed that a single peak as shown in the pattern of Fig. 1 is observed, it is now possible to produce a stable APM slurry where flocculation does not take place.

To be more specific, in order to adjust the above described APM particles to about 3 µm at the largest in terms of a median diameter, the one-step crystallization crystals of APM (i.e., the APM crystals obtained by (in) one crystallization) or the two-step crystallization crystals of APM (i.e., the APM crystals obtained from APM crystals prepared by further crystallization of the crude crystals of APM obtained by one crystallization (one-step crystallization crystals)) are ground by a conventional grinding means (such as a jet mill) to adjust the particle size of the APM to about 3 µm at the largest in terms of a median diameter.

Incidentally, with regard to the drying of the crystals after the crystallization, it is desirable to carry out that by a gas stream drier which will be mentioned later. Alternatively, there is a method where the crystals of about 3 µm are crystallized and then dried by such a drier whereby the grinding step described above is omitted.

After that, when the slurry prepared in such a manner that the particle size of APM is about 3 to 6 µm in terms of a median diameter is used as a starting substance, there are many cases showing a single peak as in the pattern of Fig. 1 and, therefore, the slurry is prepared so as not to mix the foams as same as in the above case and it is confirmed that a single peak as in the pattern of Fig. 1 is observed whereby it is possible to manufacture a stable APM slurry causing no flocculation. The crystallization condition can be adjusted for obtaining the particles within the above described particle size range or, when the crystallized crystals are outside the above described particle size range, it is possible to further adjust the particle size using a common grinding means so as to make the particle size to be within the above described range. The above crystallized crystals may be dried under ordinary conditions the same as in the case which will be mentioned later or, preferably, under a condition where damage of the crystals is little. More preferably, they are classified and obtained by drying and grinding (crushing) by a gas stream drier. An APM slurry is prepared using the particles obtained as such where the particle size of APM is about 3 to 6 µm in terms of a median diameter and mixing of foams is prevented the same as above whereupon a single peak as in the pattern of Fig. 1 is obtained with a considerably high possibility.

In the case of the slurry which is prepared in such a manner that the particle size of APM is about 6 to 10 µm in terms of a median diameter, the crystallization condition is controlled, for example, so as to give the above described particle size range and the one-step crystallization crystals of APM may be collected and dried under ordinary conditions or, preferably, under a condition where damage of the crystals is little. When an APM slurry where the particle size of APM is about 6 to 10 µm in terms of a median diameter is prepared using the particles which are classified and prepared by drying such as natural drying or by drying using a fluidized drier, conical drier, shelf drier or gas stream drier (such as Micron Drier manufactured by Hosokawa Micron K. K.) or, particularly, by drying and/or crushing using a gas stream drier and mixing of foams is prevented the same as above, a single peak like in the pattern of Fig. 1 is observed in most cases. Accordingly, when a slurry is prepared so as not to mix the foams the same as above and it is confirmed that a single peak as in the pattern of Fig. 1 is observed, then a stable APM slurry causing no flocculation can be manufactured.

When APM particles which are obtained from the crystallized crystals using a jet mill or the like are used so as to make the aimed particle size in the manufacture of the slurry, the possibility of giving a single peak as in the pattern of Fig. 1 becomes very low.

Meanwhile, regarding the above gas stream dryer, there are several functions such as a short drying time that is advantageous for a substance sensitive to heat, that damage of the crystals is little and a continuous operation is possible, that drying is conducted by air stream whereby the simultaneous transfer of the powder is possible, etc. and, accordingly, that is particularly advantageous for drying and grinding of APM in the present invention.

In the preparation of the slurry, a method corresponding to the method achieving a single peak as in the pattern of Fig. 1 shows the same pattern of the single peak so far as the same preparing method is carried out. Therefore, when a single peak is confirmed in advance, although it is not necessary thereafter to check repeatedly whether a single peak is created by the same manufacturing step, there is still a possibility that slight variations may occur and, accordingly, it is preferred to confirm whether a single peak as in the pattern of Fig. 1 is observed for each manufacturing lot.

### (APM slurry of the present invention)

This present invention is directed to the stable APM slurry which is obtainable by the above mentioned production process. The APM slurry *per se* of the present invention may be used as a liquid sweetener such as a table top sweetener or may be used as a sweetener for foods and drinks which need to be sweetened, particularly for beverage such as soft drinks. Accordingly, such a sweetener and a product where the APM is present in a stable slurry form are covered by the APM slurry of the present invention.

### (Method for the judgement of APM slurry of the present invention)

In the method for the judgement of APM slurry of the present invention, an APM slurry is subjected to the following steps so that the stable APM slurry is selected and judged:
(a) a step where the particle size distribution of the slurry is measured by a particle size distribution meter which is able to grasp the state of particle aggregation in the slurry in a nondestructive manner, and the numbers of the peaks shown in a range of not more than 1000 µm (1000 µ m or less) are counted; and
(b) a step where, in the above step, the slurry which gives substantially a single peak is recognized as a stable APM slurry.

It is also possible to improve the advantage of the present invention by adding thereto necessary step(s) other than the above steps (a) and (b) and such method is also covered by the present invention. The APM slurry which is subjected to the method for the judgement of APM slurry of the present invention can be prepared as described in the process for the preparation of APM slurry illustrated in the above described process for the manufacture of APM slurry of the present invention. Thus, as mentioned above, a slurry which is prepared by taking the conditions such as particle size of APM particles, pH value of the slurry and concentration thereof into consideration is able to be selected as the slurry to be judged. When, at first, the particle size distribution of the slurry to be judged is measured by the particle size distribution meter described above in the above step (a), the numbers of the peak in the range of 1000 µm or less can be counted and then, when mixing of foams during the preparation of APM slurry is prevented, in the above step (b) the same as in the above a single peak is observed within a range of 1000 µm or less as in the pattern of Fig. 1 in most cases whereby that (slurry) can be recognized to be the stable APM slurry.

In the preparation of the slurry, a method corresponding to the method for the preparation thereof showing a single peak as in the pattern of Fig. 1 shows the same pattern of the single peak so far as the same preparing method is carried out as mentioned above. Therefore, when a single peak is confirmed in advance, it is not necessary thereafter to check that repeatedly and it is possible to recognize the stable APM slurry without confirming the single peak repeatedly. Such a step (method) is also covered by the method for the judgement of APM slurry of the present invention. However, since there is still a possibility that slight variations may affect the method, it is preferred to confirm whether a single peak as in the pattern of Fig. 1 is observed for each of the manufacturing lots.

Further, as mentioned above, the APM slurry which is obtained or is able to be obtained by using the method for the judgement of APM slurry of the present invention is also included in the present invention. The same as the above-mentioned APM slurry of the present invention, the stable APM slurry which is thus obtained as such may be used for various uses.

### EXAMPLES

The present invention will now be illustrated in detail by way of the following Examples although the present invention is never limited by such Examples.

### (Production of APM of various particle sizes)

### [Production Example 1]

An aqueous starting material solution (380 1; 55°C; initial concentration of APM: 4.4% by weight) where 17.7 kg of aspartame (APM) were dissolved was charged in a crystallization apparatus made of stainless steel having a diameter of 400 mm and equipped with a jacket outside and a cooling plate inside, a cooling medium of 0°C temperature was circulated in the jacket and the cooling plate and then cooling was carried out for 3 hours. After about 1 hour, the whole solution became a pseudo-solid phase. The pseudo-solid phase APM crystals were dropped into a receiving vessel equipped with a cooling coil and a stirrer, disintegrated to slurry and cooled further (cooled from 16°C to 7°C in the receiving vessel). The slurry prepared as such was filtered and dehydrated using a centrifugal separator having a diameter of 36 inches whereupon wet APM crystals containing 30% water were obtained. The wet APM crystals obtained by such a static crystallization were continuously supplied to a Micron Drier (manufactured by Hosokawa Micron K. K.) using a screw feeder. They were dried until the water content became 2.6% to give dry APM crystals having an average particle size (median diameter) of about (approximately) 20 µm.

The above was prepared and adjusted into samples of the following conditions of (a), (b) and (d) and used for the manufacture of APM slurry.

### [Production Example 2]

Aspartame (APM) hydrochloride crystals (24 kg) were dissolved in 320 l of water and adjusted to pH 2.5 using 1.3 l of 28% aqueous solution of NH4OH while heating at 36°C and stirring. After that, it was heated at 65.5°C and adjusted to pH 4.9 with 3.0 l of 28% aqueous solution of NH4OH. The aqueous solution containing 4.9 g/dl of α-APM was transferred to a cylindrical crystallizing tube of 400 mm inner diameter and 3000 mm of full length equipped with a jacket having no stirring device and then cooling water of -5°C was flown into the jacket for 3.5 hours. The bottom of the crystallizing tube was opened and the solution was transferred to a crystallizing vessel equipped with a stirrer and continuously cooled with stirring for one night until the temperature became 5°C. The resulting slurry (350 1) was filtered and dehydrated using a centrifugal separator having a diameter of 36 inches to give wet crystals of APM (wet APM crystals) containing 38% of water. The wet APM crystals prepared by such a static crystallizing method were continuously supplied to a Micron Drier (manufactured by Hosokawa Micron K. K.) using a screw feeder. They were dried until the water content became 2.7% to give dry crystals of APM having an average particle size (median diameter) of about 6 µm.

The above was prepared and adjusted into the sample of the following condition of (c) and used for the manufacture of APM slurry.

### (Preparation and adjustment of APM particles)

Particles were prepared and adjusted as per the following conditions (a) to (d) using the above-manufactured APM and used for the manufacture of APM slurry:
(a) those of about 3 µm ground for adjusting the particle size (sample 1);
(b) those of about 6 µm ground for adjusting the particle size (sample 2);
(c) those of about 6 µm where no particle size adjustment was carried out (sample 3); and
(d) those of about 20 µm where no particle size adjustment was carried out (sample 4).

Incidentally, the particle size of APM was measured in terms of a median diameter using a particle size distribution meter LA920 (manufactured by Horiba K.K.) equipped with a kit for the measurement of dry substance (powder).

Meanwhile, in the above-described adjustment of particle size, APM was ground using a jet mill (STJ-475 manufactured by Seishin Kigyo K. K.).

### (Production of APM slurry)

APM slurry was produced according to the compounding formulations of the following Table 1 using the above-prepared and adjusted APM (samples 1 to 4).

**Table 1**

| Compounding Composition of APM Slurry (unit: g) | |
|---|---|
| Ingredients | Compounding Amounts |
| ① APM | 16.000 |
| ②D-Sorbitol | 202.400 |
| ③CMC sodium (*1) | 0.020 |
| ④ Methyl cellulose | 0.100 |
| ⑤ Lecithin (*2) | 0.100 |
| ⑥ NaCl | 0.035 |
| ⑦ 10% HCl | 1.800 |
| ⑧ Deionized water | 28.200 |
| ⑨ Sodium benzoate | 0.400 |
| Total | 249.055 |
| (*1) manufactured by Tokyo Kasei Kogyo K. K. (*2) manufactured by Taiyo Kagaku K. K. | |

APM and D-sorbitol were stirred and mixed (at 300 rpm for 15 minutes) so as not to mix foams therewith and then CMC sodium (carboxymethyl cellulose sodium), methyl cellulose, lecithin, NaCl, 10% HCl and deionized water (ion exchanged water) which were previously mixed and dissolved were added thereto. After that, sodium benzoate was added thereto and the mixture was stirred and mixed again (at 300 rpm for 15 minutes) to give an APM slurry (samples 1 to 4). The pH value of the resulting slurry was 4.0.

### (Evaluation of physical property by static preservation)

The resulting slurry was statically preserved in a 50-ml graduated cylinder for 3 weeks at ambient temperature and the result is shown in Table 2.

**Table 2**

| Result of Evaluation of Physical Property | |
|---|---|
| Sample Nos. | After Preserved for 3 Weeks |
| 1 | stable |
| 2 | flocculation generated |
| 3 | stable |
| 4 | particles precipitated |

### (Evaluation of physical property by Lasentec D600L [FBRM])

Immediately after the manufacture of the samples of the slurry of samples 1 to 3, their particle size distributions of APM particles were measured by a Lasentec D600L [FBRM] which is an inline type of monitoring system manufactured by Lasentec. The results are shown in Fig. 1 and Fig. 2.

The APM slurries of samples 1 and 3 showed a pattern having a single peak as shown in Fig. 1 while the APM slurry of sample 2 showed a pattern having a plurality of peaks as shown in Fig. 2.

It is noted from the above that a stable slurry where APM particles are not flocculated in the APM slurry but are homogeneously dispersed shows a pattern of Fig. 1, i.e. a single peak within a range of 1000 µm or less. It is apparent from the above that the APM slurry showing substantially a single peak as in the pattern of Fig. 1 is stable. On the other hand, it was confirmed that the APM slurry showing a plurality of peaks as in Fig. 2 was flocculated during the preservation (storage) and was unstable. Therefore, in accordance with the present invention, the slurry is prepared so as to give substantially a single peak and, therefore, a stable APM slurry can be provided.

### Effect of the Invention:

The present invention provides a stable APM slurry where the precipitating property and the flocculation (physical preservation stability) are improved and microbiological preservation stability is maintained and also provides a method for the judgement thereof. Accordingly, the present invention is very useful in industry particularly in the field of manufacture and utilization of products where a sweetener and APM are present in a form of stable slurry.

In accordance with the present invention, it is possible to provide a more stable dispersed product (APM slurry) in a stable manner even after preservation of the product for a long period from its manufacture and it is also possible to previously judge the stability of such a dispersed product.

## Claims

1. A process for producing a stable APM slurry which comprises adjusting the particle size distribution of an APM slurry in an edible dispersing medium in such way that its measurement by a particle size distribution meter, which is able to measure the state of particle aggregation in the slurry in a nondestructive manner in a pH region suitable for the microbiological preservation of food, results in a single peak within the range of 1000 µm or less, and adjusting the particle size of APM in the APM slurry to 10 µm at the largest in terms of the median diameter.

2. The process according to claim 1, wherein the pH region suitable for microbiological preservation of food is pH 4 or lower.

3. The process according to claim 1, wherein the pH region suitable for microbiological preservation of food is from pH 4 to 2.

4. The process according to any of the claims 1 to 3, wherein the particle size of APM is 6 µm, preferably 3 µm at the largest in terms of the median diameter.

5. The process according to any of claims 1 to 4, wherein particles of the APM are dried and crushed by a gas stream dryer and then the particle size thereof is adjusted or is not adjusted.

6. A stable APM slurry which is obtainable by the process according to any of claims 1 to 5.

7. A method for the determination of the stability of a stable APM slurry which comprises subjecting an APM slurry to the following steps:
(a) a step where, in case the particle size distribution of the slurry is measured by a particle size distribution meter which is able to measure the state of particle aggregation in the slurry in a nondestructive manner, number of the peaks given in the range of not more than 1000 µm is counted; and
(b) a step where an APM slurry that which gives substantially a single peak in the above step (a) is considered to be a stable APM slurry.

8. The method according to claim 7, wherein the APM slurry is prepared in a pH region which is suitable for the microbiological preservation of food.

9. The method according to claim 7 or 8, wherein the APM slurry is prepared in such a manner that the particle size of APM in the APM slurry is made 10 µm at the largest in terms of a median diameter.

10. A stable APM slurry which is obtainable by applying the method according to any of claims 7 to 9.
